(19) Europäisches Patentamt
European Patent Office
Office européen des brevets

(11) Numéro de publication: **0 132 190**
**B1**

(12) # FASCICULE DE BREVET EUROPÉEN

(45) Date de publication du fascicule du brevet: **13.01.88**

(21) Numéro de dépôt: **84401447.2**

(22) Date de dépôt: **06.07.84**

(51) Int. Cl.⁴: **C 07 C 133/12**, C 07 D 233/52, C 07 D 233/50, C 07 D 233/24, A 61 K 31/415, A 61 K 31/155

(54) **Nouvelles amidines, leur procédé de préparation et leur application en thérapeutique.**

(30) Priorité: **13.07.83 FR 8311717**

(43) Date de publication de la demande: **23.01.85 Bulletin 85/4**

(45) Mention de la délivrance du brevet: **13.01.88 Bulletin 88/2**

(84) Etats contractants désignés: **AT BE CH DE FR GB IT LI LU NL SE**

(56) Documents cité:
**EP-A-0 035 393**
**EP-A-0 043 659**
**EP-A-0 061 924**
**FR-A-1 332 543**
**FR-A-1 549 283**
**FR-M-8 343**

**CHEMICAL ABSTRACTS, vol. 70, no. 11, 17 mars 1969, réf. no. 47453k, page 338, Columbus, Ohio (US);**

**Le dossier contient des informations techniques présentées postérieurement au dépôt de la demande et ne figurant pas dans le présent fascicule.**

(73) Titulaire: **LABORATOIRES CHAUVIN- BLACHE Société dite:, 104 Rue de la Galéra, F-34000 Montpellier (FR)**

(72) Inventeur: **Battais, Elisabeth, Les Pyramides d'Alco Bât. A, No. 6 856 Rue d'Alco, F-34100 Montpellier (FR)**
Inventeur: **Sincholle, Daniel 343 Chemin de la Trémoulette, Saint- Clément- la- Rivière, F-34980 Saint- Gely- du- Fesc (FR)**
Inventeur: **Coquelet, Claude, 113 Rue des Lilas, F-34980 Saint- Gely- du- Fesc (FR)**
Inventeur: **Bonne, Claude, 12 bis Avenue Aristide Briand, F-94360 Bry- sur- Marne (FR)**

(74) Mandataire: **Polus, Camille, c/o Cabinet Lavoix 2, Place d'Estienne d'Orves, F-75441 Paris Cedex 09 (FR)**

**EP 0 132 190 B1**

Il est rappelé que: Dans un délai de neuf mois à compter de la date de publication de la mention de la délivrance du brevet européen toute personne peut faire opposition au brevet européen délivré, auprés de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention concerne de nouvelles amidines ayant une activité anti-hypertensive et qui peuvent être utilisées notamment dans le traitement des hypertensions oculaires telles que le glaucome.

Les phénomènes d'hypertension oculaire sont liés principalement à un déséquilibre entre le débit de formation et le débit d'écoulement de l'humeur aqueuse.

L'humeur aqueuse est produite par le corps ciliaire; elle passe dans la chambre postérieure, traverse la pupille et arrive dans la chambre antérieure où elle gagne la périphérie pour atteindre l'angle iridocornéen. Le sommet de cet angle est occupé par un tissu conjonctif à larges mailles: le trabeculum. A ce niveau, l'humeur aqueuse s'infiltre entre les mailles de celui-ci, est collectée dans le canal de Schlemm, puis gagne par les veines aqueuses la circulation veineuse épisclérale et enfin la circulation générale.

Chez les sujets atteints de glaucome, le taux d'élimination de l'humeur aqueuse est réduit; il en résulte une accumulation d'humeur aqueuse et une hypertension oculaire.

En l'absence de traitement, cette pression élevée peut interférer avec la circulation sanguine au niveau des fibres nerveuses de la rétine et du nerf optique entraînant la destruction du nerf optique et la cécité.

Parmi les substances couramment utilisées dans le traitement du glaucome, on peut citer à titre d'exemples :
- les cholinergiques, tels la pilocarpine;
- les inhibiteurs de l'anhydrase carbonique, tels l'acétazolamide;
- les adrénergiques, tels l'adrénaline, agoniste α et β non sélectif et le timolol, sympatholytique β non sélectif.

Différentes études ont montré que l'administration par voie topique de substances stimulant les récepteurs adrénergiques de type $\alpha_2$ ,telles la clonidine et l'α -méthyl adrénaline, entraînait une diminution de la pression intraoculaire (ALLEN R.C.,Invest. Ophthalmol.,15, (10), 815, 1976 : KRIEGLSTEIN G.K.,Invest. Ophthalmol. Vis. Sci. 17 (2), 149, 1978 ; HODAPP E.,Arch. Ophthalmol.,99, 1208, 1981 ; LANGHAM M.E. Invest.Ophthalmol.Vis.Sci.ARVO suppl.,22,230,1982).

Par son action sur le système nerveux central la clonidine présente des effets secondaires gênants à côté de son action sur la pression intraoculaire.

Par ailleurs, dans les BSM 8175 et 7689, on a décrit des amidines de type benzylidène amino guanidine pour le traitement général de l'hypertension artérielle. Ces composés présentent une composante sédative par leur action sur le système nerveux central.

EP-A-081 924 décrit l'utilisation de 2-(phénylimino-trisubstitué) imidazolines pour abaisser la pression intraoculaire. Ces composés ont toutefois un effet non négligeable sur le système nerveux central.

FR-A-1 549 283 décrit des 2-anilinométhylimidazolines substituées par des halogènes ayant un effet hypotenseur.

C.A. 70, 11, p. 338, n° 47453k décrit des 2-arylamino-2-imidazolines ayant notamment des effets antihypertenseur et sédatif.

FR-M 8343 décrit l'utilisation de benzylidène aminoguanidines comme hypotenseurs.

La présente invention est basée sur la découverte de nouvelles amidines ayant une activité adrénergique $\alpha_2$ stimulante et pratiquement dépourvues d'action sur le système nerveux central, qui peuvent être utilisées comme antihypertenseurs, notamment dans le domaine oculaire.

La présente invention a ainsi pour objet des composés de formule

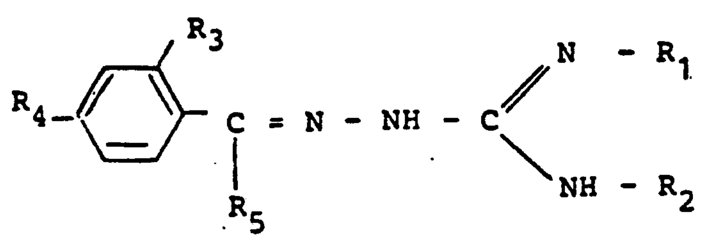

(II)

dans laquelle

$R_1$ et $R_2$ représentent chacun un atome d'hydrogène ou forment ensemble un radical bivalent éthylène.

$R_3$ représente un atome d'halogène, un groupe méthyle , un groupe méthoxy , un groupe amino, un groupe acétamido ou un groupe sulfonamido,

$R_4$ représente un groupe hydroxy,

un groupe amino, un groupe acétamido ou un groupe sulfonamido, et

$R_5$ représente un atome d'hydrogène ou un groupe méthyle, ainsi que leurs sels d'addition avec des acides pharmaceutiquement acceptables.

La présente invention a également pour objet des compositions thérapeutiques contenant à titre de principe actif un composé de formule II ou l'un de ses sels d'addition avec des acides pharmaceutiquement acceptables.

Dans la définition ci-dessus, on désigne par "sels d'addition avec des acides pharmaceutiquement acceptables", les sels qui possèdent les propriétés biologiques des bases libres, sans avoir d'effet indésirable. Ces sels peuvent être notamment ceux formés avec des acides minéraux tels que l'acide chlorhydrique, l'acide

bromhydrique, l'acide sulfurique, l'acide nitrique, l'acide phosphorique, des sels métalliques acides, tels que l'orthophosphate disodique et le sulfate monopotassique et des acides organiques, tels que l'acide formique, l'acide acétique, l'acide propionique, l'acide glycolique, l'acide oxalique, l'acide fumarique, l'acide citrique, l'acide malique, l'acide tartrique et l'acide pamoïque.

Le terme "halogène" désigne le chlore, le brome ou l'iode.

Pour préparer les composés de formule II, on peut faire réagir un aldéhyde ou une cétone de formule

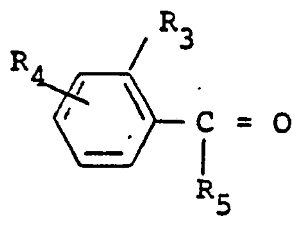

(VI)

dans laquelle $R_3$, $R_4$ et $R_5$ ont les significations données ci-dessus, avec un composé de formule

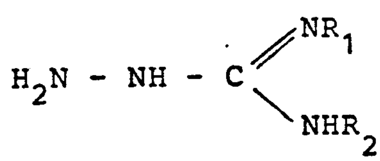

(VII)

dans laquelle $R_1$ et $R_2$ ont les significations données ci-dessus, ou l'un de ses sels.

En particulier, dans le cas où $R_1$ et $R_2$ ne forment pas cycle, la réaction peut être effectuée en solution dans du n-butanol en présence d'un acide concentré tel que l'acide chlorhydrique, l'eau du sousproduit étant éliminée par voie azéotropique.

On peut également faire réagir un composé de formule VII sur la cétone ou l'aldéhyde de formule VI par chauffage dans la pyridine.

Dans le cas où $R_1$ et $R_2$ forment un cycle, les réactions connues par l'homme de l'art pour la synthèse des hydrazones peuvent être utilisées. Ainsi, on peut faire réagir l'aldéhyde ou la cétone de formule VI avec un composé cyclique de formule VII en portant à reflux des quantités équimolaires des composés en solution alcoolique, en présence d'un catalyseur acide tel que l'acide acétique ou l'acide chlorhydrique. On peut également faire réagir dans les mêmes conditions un sel de composé cyclique de formule VII, tel que le bromhydrate ou le chlorhydrate.

Les sels d'addition avec des acides pharmaceutiquement acceptables peuvent être préparés de manière classique par réaction des bases libres avec un acide ou un sel, notamment en solution dans un alcool.

On donnera ci-après dans le Tableau I des exemples de composés selon la présente invention. Ces composés ont été préparés selon les modes opératoires suivants:

**Mode opératoire A** -

Des quantités équimolaires d'aldéhyde ou de cétone VI et de bromhydrate d'hydrazino-2 imidazoline sont portées à reflux dans du méthanol en présence d'une quantité catalytique d'acide acétique pendant une durée qui peut varier de 15 minutes à 2 heures. Après refroidissement, l'hydrazone formée est isolée après barbotage d'ammoniac dans la solution méthanolique, suivi de l'évaporation sous vide du méthanol. Le produit brut est transformé en chlorhydrate par barbotage d'acide chlorhydrique gazeux dans une solution alcoolique de la base. Le sel brut est recristallisé dans un mélange méthanol-éther diéthylique.

**Mode opératoire B** -

0,1 mole d'aldéhyde ou de cétone VI, 0,1 mole de bicarbonate d'aminoguanidine, 10 ml d'acide chlorhydrique concentré et 50 ml de n-butanol sont portés à reflux dans un ballon surmonté d'un piège Dean Stark pendant 3 heures. Après évaporation sous vide du solvant, le résidu est trituré dans une solution d'éther éthylique saturée en acide chlorhydrique gazeux. Les cristaux bruts obtenus sont recristallisés dans un mélange méthanol-éther diéthylique.

**TABLEAU I**

**COMPOSES DE FORMULE II**

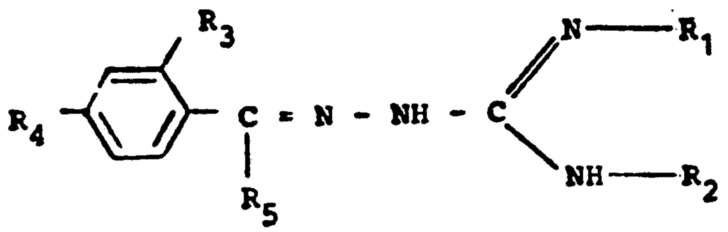

| Composé | $R_1$,$R_2$ | $R_3$ | $R_4$ | $R_5$ | Mode opératoire | Rendement (%) | Fusion (°C) du chlorhydrate |
|---|---|---|---|---|---|---|---|
| 1 | $-CH_2-CH_2$ | $CH_3$ | OH | H | A | 64 | > 255 |
| 2 | " | $CH_3$ | $NH_2$ | H | A | 70 | > 255 |
| 3 | " | $CH_3$ | OH | $CH_3$ | A | 62 | 175-7 |
| 4 | " | $OCH_3$ | OH | H | A | 63 | 198 (dec) |
| 5 | " | $OCH_3$ | $NH_2$ | H | A | 54 | 207-9 |
| 6 | " | $OCH_3$ | OH | $CH_3$ | A | 70 | 166-8 |

**TABLEAU I** (suite)

| Composé | $R_1$ | $R_2$ | $R_3$ | $R_4$ | $R_5$ | Mode opératoire | Rendement (%) | Fusion (°C) du chlorhydrate |
|---|---|---|---|---|---|---|---|---|
| 7 | H | H | $CH_3$ | OH | H | B | 51 | 205-7 |
| 8 | H | H | $CH_3$ | $NH_2$ | H | B | 62 | 254-256 |
| 9 | H | H | $OCH_3$ | OH | H | B | 45 | 219-221 |
| 10 | H | H | $CH_3$ | OH | $CH_3$ | B | 50 | 168-70 |
| 11 | H | H | $OCH_3$ | OH | $CH_3$ | B | 55 | 193-195 |

On donnera ci-après des résultats des études biochimiques, pharmacologiques et toxicologiques mettant en évidence les propriétés des composés de formule II.

**1. Affinité pour le récepteur adrénergique $\alpha_2$ plaquettaire**

Les composés de formule I ont été testés sur le récepteur adrénergique plaquettaire de lapin qui est exclusivement de type $\alpha_2$ (MICHEL T., Nature, 288, (5792) 709, 1980).

L'affinité de ces composés pour le récepteur $\alpha_2$ est déterminée en mesurant l'inhibition de la liaison spécifique de la dihydroergocryptine (DHE) tritiée par les composés testés.

Les lapins utilisés sont des lapins albinos mâles Néo-Zélandais.

On prélève en intracardiaque du sang sur ACD (anticoagulant constitué par une solution aqueuse contenant 0,27 g d'acide citrique, 0,5 g de citrate trisodique et 0,02 g de glucose pour 20 ml d'eau) à raison de 1 volume d'ACD pour 6 volumes de sang.

Le sang est centrifugé 10 minutes à 280g et le surnageant (PRP) est collecté. Le PRP est centrifugé 15 minutes à 2000g.

Le culot plaquettaire est lavé deux fois dans le tampon I (Tris 50 mM, NaCl 150 mM, EDTA 20 mM, pH 7,5) puis remis en suspension dans le tampon II (Tris 50 mM, EDTA 5 mM, pH 7,5).

Cette suspension est congelée à -40°C, puis décongelée; le processus est répété deux fois. La suspension est ensuite homogénéisée, puis centrifugée 10 minutes à 40.000g, à 4°C.

Le culot est lavé dans le tampon II puis remis en suspension dans ce même tampon de telle sorte que la concentration finale en protéines soit de l'ordre de 1,5 mg/ml.

Les essais d'inhibition sont effectués dans un volume final de 0,2 ml de tampon II contenant la DHE tritiée (7 nM) et des concentrations variables de composé testé.

La réaction est initiée en ajoutant 0,2 mg de protéines et poursuivie pendant 30 minutes à 25°C.

L'incubation est arrêtée en ajoutant 2 ml de tampon II et en filtrant rapidement ce mélange sous vide sur filtres Whatman GF/C. Les filtres sont lavés rapidement par du tampon II et la radioactivité liée au filtre est mesurée en scintillation liquide.

La dose nécessaire pour inhiber 50 pour cent de la liaison de la DHE tritiée ($EC_{50}$) est calculée pour chacun

des composés testés.

Les résultats sont donnés dans le Tableau suivant.

**TABLEAU II**

| Composé | $EC_{50}$ ($10^{-6}$ M) |
|---|---|
| 1 | 3 |
| 2 | 10 |
| 3 | 5,6 |
| 4 | 3 |
| 5 | 14 |
| 6 | 2,5 |
| 7 | 6,3 |
| 8 | 32 |
| 9 | 8 |
| 10 | 11 |
| 11 | 1,3 |

**2. Activité hypotensive chez le lapin après administration topique**

Les expérimentations ont été faites sur des lapins mâles Néo-Zélandais de 2 kg, nourris et abreuvés "ad libitum".

Les animaux sont habitués quelques jours à l'animalerie, conditionnés à la stabulation en boîte à contention et à la prise de mesure de pression intraoculaire, afin d'éviter des écarts de tension dus au stress de la manipulation.

La pression intraoculaire est mesurée à l'aide d'un pneumatonographe à aplanation ALCON, calibré à l'aide d'une cornée de lapin isolée, sous pression d'eau.

Les composés ont été testés en solution aqueuse, isotonique et à pH 7. 25µl d'une solution à 0,5 % sont instillés dans un oeil et la pression intraoculaire mesurée au cours du temps.

Le Tableau ci-après donne l'abaissement maximum de la pression intraoculaire (Δ max) par rapport à la valeur de base au temps zéro (moment de l'instillation), le temps correspondant à cet effet maximum (t), ainsi que la durée totale de l'effet.

**TABLEAU III**

| Composé | Δ max (mm Hg) | t (mn) | Durée de l'effet (mn) |
|---|---|---|---|
| 1 | -3,12 ± 2,21 | 60 | 360 |
| 2 | -4,12 ± 0,95 | 60 | 300 |
| 3 | -1,83 ± 1 | 30 | 300 |
| 4 | -2,5 ± 0,57 | 60 | 400 |
| 5 | -3,9 ± 0,5 | 120 | 360 |
| 7 | -0,25 ± 1,7 | 60 | - |

Dans les mêmes conditions expérimentales, la clonidine (2,6-dichloro-anilino-2-imidazoline) ainsi que la (2,6-dichloro-benzilidène-hydrazino)-2-imidazoline (composé de l'exemple 1 du BSM 8175) provoquent une hypertension dans l'oeil traité et un abaissement concomitant de la pression dans l'oeil contralatéral, ce dernier effet mettant en évidence une action au niveau du système nerveux central. Ce phénomène est absent avec les composés de formule I.

**3. Toxicité aiguë**

Les souris utilisées sont des souris mâles de souche Swiss (NMRI-Han), d'un poids moyen de 25 g.

L'étude de la toxicité aiguë à été réalisée par voie intrapéritonéale.

Les composés ont été injectés en solution aqueuse à trois doses : 50, 100 et 200 mg/kg.

Le tableau ci-après donne les résultats obtenus avec les composés de formule II , après un délai d'observation de 48 heures.

Ces résultats sont exprimés en nombre d'animaux morts sur le nombre total d'animaux traités.

**TABLEAU IV**

| Composé | 50 mg/kg | 100 mg/kg | 200 mg/kg |
|---|---|---|---|
| 1 | - | 0/3 | 0/3 |
| 2 | - | 0/3 | 3/3 |
| 3 | - | 0/3 | 3/3 |
| 4 | - | 0/3 | 0/3 |
| 5 | - | 0/3 | ND |
| 7 | - | 0/3 | 3/3 |
| 8 | - | 0/3 | 3/3 |
| 9 | 0/3 | 3/3 | 3/3 |
| 10 | - | 0/3 | 2/3 |

Les compositions thérapeutiques selon l'invention peuvent être administrées à l'homme ou aux animaux par voie topique, orale ou parentérale.

Elle peuvent être sous la forme de préparations solides, semi-solides ou liquides. Comme exemples, on peut citer les comprimés, les gélules, les suppositoires, les solutions ou suspensions injectables, les pommades, les collyres huileux ou aqueux, les collutoires, les solutions nasales et otologiques, ainsi que les formes retard. Une forme préférée est constituée par les collyres et les implants ophtalmiques.

Dans ces compositions, le principe actif est généralement mélangé avec un ou plusieurs excipients pharmaceutiquement acceptables habituels bien connus de l'homme de l'art.

Les compositions thérapeutiques administrables par voie topique peuvent contenir notamment de 0,1 à 5 % en poids de principe actif.

Les compositions thérapeutiques administrables par voie orale ou parentérale peuvent contenir notamment de 1 à 60 % en poids de principe actif.

La quantité de principe actif administrée dépend évidemment du patient qui est traité, de la voie d'administration et de la sévérité de la maladie. Cependant, pour l'administration par voie orale ou parentérale, on peut administrer environ de 0,1 à 10 mg/kg/jour, soit chez un homme de 100 kg de 10 mg à 1 g/jour.

**Revendications** pour les Etats contractants BE CH DE FR GB IT LI LU NL SE

1. Composés de formule

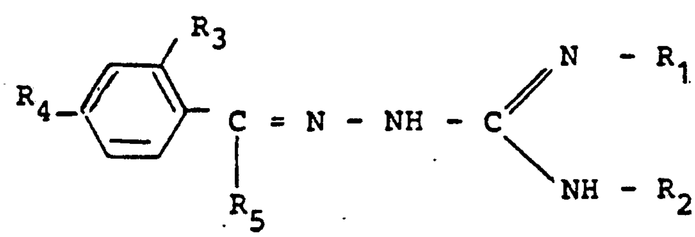

(II)

dans laquelle

$R_1$ et $R_2$ représentent chacun un atome d'hydrogène ou forment ensemble un radical bivalent éthylène.

$R_3$ représente un atome d'halogène, un groupe méthyle, un groupe méthoxy, un groupe amino, un groupe acétamido ou un groupe sulfonamido,

$R_4$ représente un groupe hydroxy, un groupe amino, un groupe acétamido ou un groupe sulfonamido, et

$R_5$ représente un atome d'hydrogène ou un groupe méthyle, ainsi que leurs sels d'addition avec des acides pharmaceutiquement acceptables.

2. Composés selon la revendication 1, caractérisés en ce que $R_3$ représente un groupe méthyle ou méthoxy et $R_4$ représente un groupe hydroxy.

3. Composés selon la revendication 2, caractérisés en ce que $R_3$ représente un groupe méthyle.

4. Composés selon la revendication 3, caractérisés en ce que $R_1$ et $R_2$ forment ensemble un radical bivalent éthylène et $R_5$ représente un atome d'hydrogène.

5. Procéde de préparation d'un composé selon la revendication 1, caractérisé en ce que l'on fait réagir un aldéhyde ou une cétone de formule

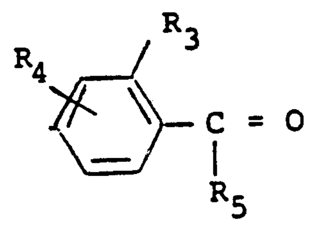

(VI)

dans laquelle $R_3$, $R_4$ et $R_5$ ont les significations données à la revendication 1, avec un composé de formule

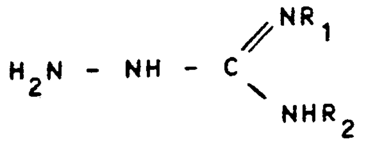

(VII)

dans laquelle $R_1$ et $R_2$ ont les significations données à la revendication 1, ou l'un de ses sels.

6. Composition thérepautique, caractérisée en ce qu'elle contient à tire de principe actif un composé selon l'une quelconque des revendications 1 à 4.

7. Composition thérapeutique selon la revendication 6 sous forme d'un collyre.

8. Composition selon la revendication 7, caractérisé en ce qu'elle comprend de 0,1 à 5 % en poids de principe actif.

9. Composition thérapeutique selon la revendication 6, sous forme d'implant ophtalmique.

**Revendications** pour l'Etat Contractant AT

1. Procédé de préparation de composés de formule

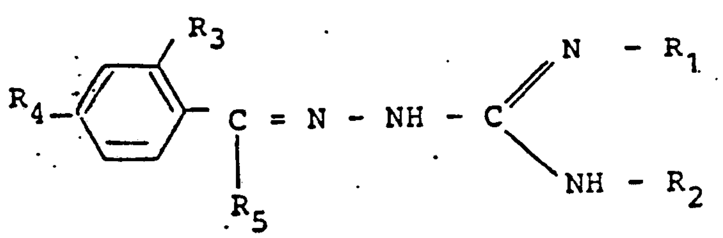

(II)

dans laquelle

$R_1$ et $R_2$ représentent chacun un atome d'hydrogène ou forment ensemble un radical bivalent éthylène,

$R_3$ représente un atome d'halogène, un groupe méthyle, un groupe méthoxy, un groupe amino, un groupe acétamido ou un groupe sulfonamido,

$R_4$ représente un groupe hydroxy,

un groupe amino, un groupe acétamido ou un groupe sulfonamido, et

$R_5$ représente un atome d'hydrogène ou un groupe méthyle,

ainsi que de leurs sels d'addition avec des acides pharmaceutiquement acceptables,

caractérisé en ce que l'on fait réagir un aldéhyde ou une cétone de formule

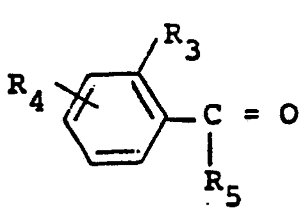

(VI)

dans laquelle $R_3$, $R_4$ et $R_5$ ont les significations données ci-dessus avec un composé de formule

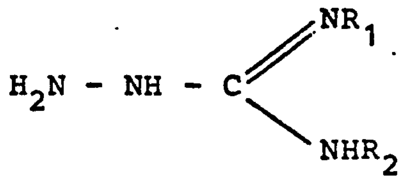

(VII)

dans laquelle $R_1$ et $R_2$ ont les significations données ci-dessus, ou l'un de ses sels.

2. Procédé selon la revendication 1, caractérisé en ce que $R_3$ représente un groupe méthyle ou méthoxy et $R_4$ représente un groupe hydroxy.

3. Procédé selon la revendication 2, caractérisé en ce que $R_3$ représente un groupe méthyle.

4. Procédé selon la revendication 3, caractérisé en ce que $R_1$ et $R_2$ forment ensemble un radical bivalent éthylène et $R_5$ représente un atome d'hydrogène.

5. Procédé de préparation d'une composition pharmaceutique caractérisé en ce que l'on met un composé de formule

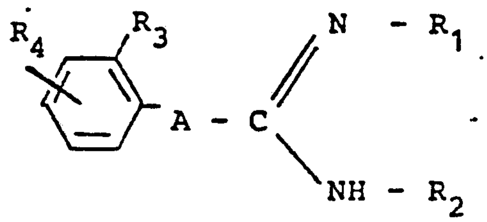

(I)

dans laquelle

$R_1$ et $R_2$ représentent chacun un atome d'hydrogène ou forment ensemble un radical bivalent éthylene,

$R_3$ représente un atome d'halogène, un groupe méthyle, un groupe méthoxy, un groupe amino, un groupe acétamido ou un groupe sulfonamido,

$R_4$ représente un groupe hydroxy,

un groupe amino, un groupe acétamido ou un groupe sulfonamido, et

$R_5$ représente un atome d'hydrogène ou un groupe méthyle,

ou l'un de ses sels d'addition avec des acides pharmaceutiquement acceptables, sous une forme convenant pour l'administration thérapeutique.

**Patentansprüche** (für die Vertragsstaaten BE CH DE FR GB IT LI LU NL SE

1. Verbindungen der Formel

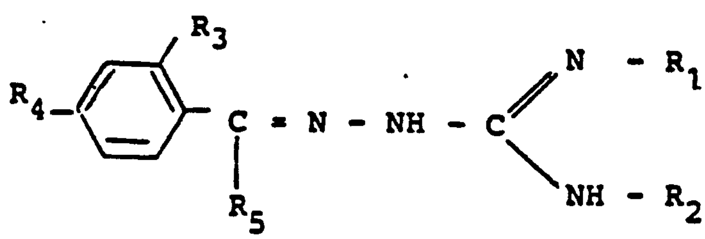

(II)

in der $R_1$ und $R_2$ jeweils ein Wasserstoffatom sind oder zusammen einen zweiwertigen Ethylenrest bilden,

$R_3$ ein Halogenatom, eine Methylgruppe, eine Methoxygruppe, eine Aminogruppe, eine Acetamidogruppe oder eine Sulfonamidogruppe darstellt,

$R_4$ eine Hydroxylgruppe, eine Aminogruppe, eine Acetamidogruppe oder eine Sulfonamidogruppe ist, und

$R_5$ ein Wasserstoffatom oder eine Methylgruppe darstellt, sowie ihre Additionssalze mit pharmazeutisch unbedenklichen Säuren.

2. Verbindungen nach Anspruch 1, dadurch gekennzeichnet, daß $R_3$ eine Methylgruppe oder Methoxygruppe und $R_4$ eine Hydroxylgruppe ist.

3. Verbindungen nach Anspruch 2, dadurch gekennzeichnet, daß $R_3$ eine Methylgruppe ist.

4. Verbindungen nach Anspruch 3, dadurch gekennzeichnet, daß $R_1$ und $R_2$ zusammen einen zweiwertigen Ethylenrest bilden und $R_2$ ein Wasserstoffatom ist.

5. Verfahren zur Herstellung einer Verbindung nach Anspruch 1, dadurch gekennzeichnet, daß man einen

Aldehyd oder ein Keton der Formel

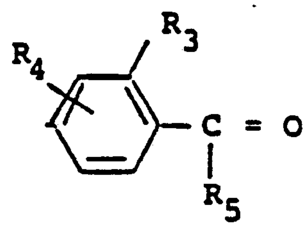

(VI)

in der $R_3$, $R_4$ und $R_5$ die in Anspruch 1 genannten Bedeutungen haben mit einer Verbindung der Formel

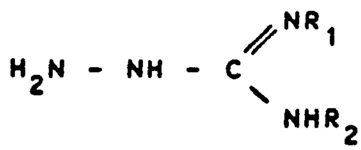

(VII)

in der $R_1$ und $R_2$ die in Anspruch 1 genannten Bedeutungen haben, oder einem ihrer Salze umsetzt.

6. Therapeutische Zubereitung, dadurch gekennzeichnet, daß sie eine Verbindung nach irgendeinem der Ansprüche 1 bis 4 als Wirkstoff enthält.

7. Therapeutische Zubereitung nach Anspruch 6 in Form eines Leims.

8. Zubereitung nach Anspruch 7, dadurch gekennzeichnet, daß sie 0,1 bis 5 Gew.-% Wirkstoff enthält.

9. Therapeutische Zubereitung nach Anspruch 6 in Form eines ophthalmischen Implantats.

**Patentansprüche** für den Vertragstaat: AT

1. Verfahren zur Herstellung von Verbindungen der Formel

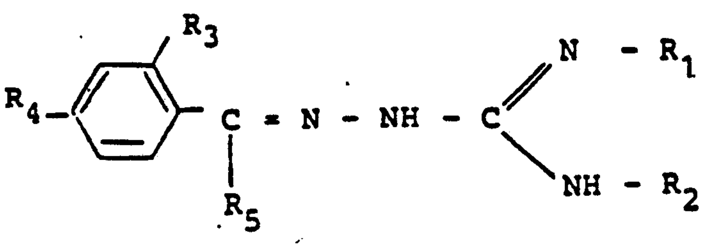

(II)

in der $R_1$ und $R_2$ jeweils ein Wasserstoffatom sind oder zusammen einen zweiwertigen Ethylenrest bilden,
$R_3$ ein Halogenatom, eine Methylgruppe, eine Methoxygruppe, eine Aminogruppe, eine Acetamidogruppe oder eine Sulfonamidogruppe darstellt,
$R_4$ eine Hydroxylgruppe, eine Aminogruppe, eine Acetamidogruppe oder eine Sulfonamidogruppe ist, und
$R_5$ ein Wasserstoffatom oder eine Methylgruppe darstellt, sowie ihrer Additionssalze mit pharmazeutisch unbedenklichen Säuren, dadurch gekennzeichnet, daß man einen Aldehyd oder ein Keton der Formel

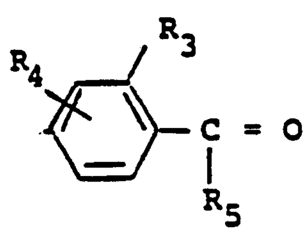

(VI)

in der $R_3$, $R_4$ und $R_5$ die vorstehend genannten Bedeutung haben, mit einer Verbindung der Formel

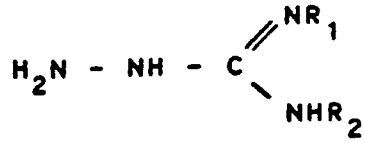

(VII)

in der $R_2$ und $R_2$ die vorstehend genannten Bedeutungen haben, oder einem ihrer Salze umsetzt.

2. Verfahren nach Anspruch 1, dadurch gekennzeichnet, daß $R_3$ eine Methylgruppe oder Methoxygruppe und $R_4$ eine Hydroxylgruppe ist.

3. Verfahren nach Anspruch 2, dadurch gekennzeichnet, daß $R_3$ eine Methylgruppe ist.

4. Verfahren nach Anspruch 3, dadurch gekennzeichnet, daß $R_1$ und $R_2$ zusammen einen zweiwertigen Ethylenrest bilden und $R_5$ ein Wasserstoffatom ist.

5. Verfahren zur Herstellung einer pharmazeutischen Verbindung, dadurch gekennzeichnet, daß man eine Verbindung der Formel

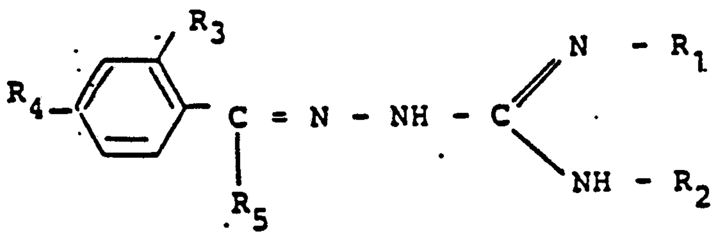

(II)

in der $R_1$ und $R_2$ jeweils ein Wasserstoffatom sind oder zusammen einen zweiwertigen Ethylenrest bilden,

$R_3$ ein Halogenatom, eine Methylgruppe, eine Methoxygruppe, eine Aminogruppe, eine Acetamidogruppe oder eine Sulfonamidogruppe darstellt,

$R_4$ eine Hydroxylgruppe, eine Aminogruppe, eine Acetamidogruppe oder eine Sulfonamidogruppe ist, und

$R_5$ ein Wasserstoffatom oder eine Methylgruppe darstellt, oder eines ihrer Additionssalze mit pharmazeutisch annehmbaren Säuren in eine zur therapeutischen Verabreichung geeignete Form bringt.

**Claims** for the contracting states BE CH DE FR GB IT LI LU NL SE

1. Compounds of formula:

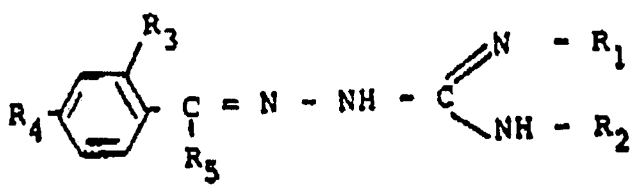

(II)

in which:

$R_1$ and $R_2$ each represent a hydrogen atom or together form a divalent ethylene radical,

$R_3$ represents a halogen atom, a methyl group, a methoxy group, an amino group, an acetamido group or a sulfonamido group,

$R_4$ represents a hydroxy group, an amino group, an acetamido group or a sulfonamido group, and

$R_5$ represents a hydrogen atom or a methyl group, and their pharmaceutically acceptable acid addition salts.

2. Compounds as claimed in claim 1, characterised in that $R_3$ represents a methyl or methoxy group and $R_4$ represents a hydroxy group.

3. Compounds as claimed in claim 2, characterised in that $R_3$ represents a methyl group.

4. Compounds as claimed in claim 3, characterised in that $R_1$ and $R_2$ together form a divalent ethylene radical and $R_5$ represents a hydrogen atom.

5. Process for the preparation of a compound as claimed in claim 1, characterised in that an aldehyde or a ketone of formula

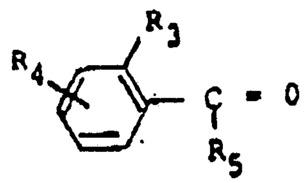

(VI)

in which $R_3$, $R_4$ and $R_5$ are as defined in claim 1, is reacted with a compound of formula

$$H_2N - NH - C(=NR_1)-NHR_2 \quad (VII)$$

in which $R_1$ and $R_2$ are as defined in claim 1, or a salt thereof.

6. Therapeutic composition, characterized in that it contains, as active ingredient, a compound as claimed in any one of claims 1 to 4.

7. Therapeutic composition as claimed in claim 6, in the form of a collyrium.

8. Composition as claimed in claim 7, characterised in that it contains from 0,1 to 5 % by weight of active principle.

9. Therapeutic composition as claimed in claim 6, in the form of an ophthalmic implant.

**Claims** for the contracting state AT

1. Process for preparing compounds of formula:

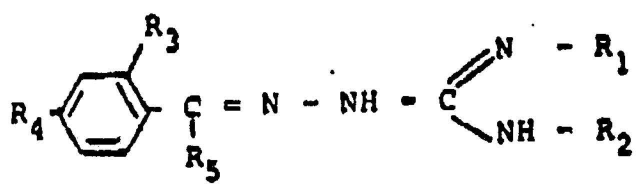

(II)

in which:

$R_1$ and $R_2$ each represent a hydrogen atom or together form a divalent ethylene radical,

$R_3$ represents a halogen atom, a methyl group, a methoxy group, an amino group, an acetamido group or a sulfonamido group,

$R_4$ represents a hydroxy group, an amino group, an acetamido group or a sulfonamido group, and

$R_5$ represents a hydrogen atom or a methyl group, and their pharmaceutically acceptable acid addition salts, characterised in that an aldehyde or a ketone of formula

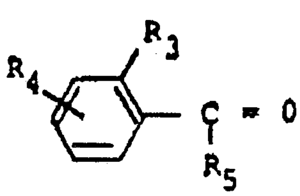

(VI)

in which $R_3$, $R_4$ and $R_5$ are as defined in claim 1, is reacted with a compound of the formula (VII):

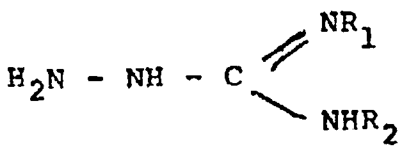

(VII)

in which $R_1$ and $R_2$ are as defined in claim 1, or a salt thereof.

2. Compounds as claimed in claim 1, characterised in that $R_3$ represents a methyl or methoxy group and $R_4$ represents a hydroxy group.

3. Compounds as claimed in claim 2, chacacterised in that $R_3$ represents a methyl group.

4. Compounds as claimed in claim 3, characterised in that $R_1$ and $R_2$ together form a divalent ethylene radical and $R_5$ represents a hydrogen atom.

5. Process for preparing a pharmaceutical composition, characterised in that a compound of formula

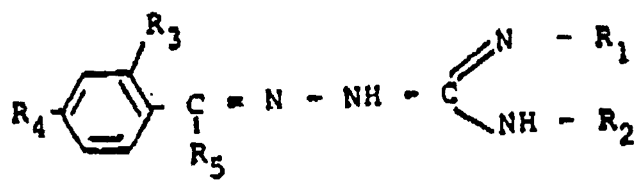

(II)

wherein

$R_1$ and $R_2$ each represent a hydrogen atom or together form a divalent ethylene radical,

$R_3$ represents a halogen atom, a methyl group, a methoxy group, an amino group, an acetamido group or a sulfonamido group,

$R_4$ represents a hydroxy group, an amino group, an acetamido group or a sulfonamido group, and

$R_5$ represents a hydrogen atom or a methyl group, or a pharmaceutically acceptable acid addition salt thereof, is put into a form suitable for therapeutic administration.